Europäisches Patentamt

European Patent Office

Office européen des brevets

(19)

(11) Veröffentlichungsnummer: **0 022 932**
**B1**

(12) **EUROPÄISCHE PATENTSCHRIFT**

(45) Veröffentlichungstag der Patentschrift:
02.11.83

(51) Int. Cl.³: **A 61 K 9/18**, A 61 K 31/135

(21) Anmeldenummer: **80103393.7**

(22) Anmeldetag: **18.06.80**

(54) Zusammensetzung mit verlängerter broncholytischer und tokolytischer Wirksamkeit, ein Verfahren zu deren Herstellung und broncholytisch und tokolytisch wirkendes Mittel.

(30) Priorität: **20.07.79 DE 2929456**

(43) Veröffentlichungstag der Anmeldung:
**28.01.81 Patentblatt 81/4**

(45) Bekanntmachung des Hinweises auf die Patenterteilung:
**02.11.83 Patentblatt 83/44**

(84) Benannte Vertragsstaaten:
**BE CH DE FR GB LI NL SE**

(56) Entgegenhaltungen:
**DE - A - 2 362 123**
**DE - A - 2 737 604**
**FR - A - 806**
**GB - A - 857 193**

**Die Akte enthält technische Angaben, die nach dem Eingang der Anmeldung eingereicht wurden und die nicht in dieser Patentschrift enthalten sind.**

(73) Patentinhaber: **CHEMIE LINZ AKTIENGESELLSCHAFT,
St. Peter-Strasse 25, A-4020 Linz (AT)**

(84) Benannte Vertragsstaaten: **BE CH FR GB LI NL SE**

(73) Patentinhaber: **Lentia Gesellschaft mit beschränkter Haftung, Arabellastrasse 4 Postfach 81 05 08,
D-8000 München 81 (DE)**

(84) Benannte Vertragsstaaten: **DE**

(72) Erfinder: **Siegl, Annemarie, Dr., Carl Boschweg 7,
A-4020 Linz (AT)**
Erfinder: **Reithmayr, Karl, Dr., Karl Rennerstrasse 4,
A-4045 Linz (AT)**
Erfinder: **Knopp, Christian, Dr., Pensionsweg 10,
A-8043 Graz (AT)**

# Zusammensetzung mit verlängerter broncholytischer und tokolytischer Wirksamkeit, ein Verfahren zu deren Herstellung und broncholytisch und tokolytisch wirkendes Mittel

Die vorliegende Erfindung betrifft Zusammensetzungen mit verlängerter broncholytischer und tokolytischer Wirksamkeit, die sich vom β₂-sympathomimetisch wirkenden Stoff N,N'-Bis-[2-(3',4'-dihydroxyphenyl)-2-hydroxyäthyl]-hexamethylendiamin (Hexoprenalin) ableiten, ein Verfahren zur Herstellung der Zusammensetzung und ein broncholytisch und tokolytisch wirkendes Mittel, das den Wirkstoff Hexoprenalin langsam und gleichmässig abgibt.

N,N'-Bis-[2-(3',4'-dihydroxyphenyl)-2-hydroxyäthyl]-hexamethylendiamin und dessen Salze sind schon seit längerer Zeit als β₂-Mimetikum und als Wirkstoff in broncholytisch wirkenden Mitteln bekannt (AT-PS 241 436). Ein Vorteil dieses Wirkstoffes liegt in den geringen Nebenwirkungen auf Herz und Blutdruck und einer relativ langen Wirkungsdauer. In der klinischen Praxis hat sich allerdings ergeben, dass eine Verlängerung der Wirkungsdauer wünschenswert wäre.

In der DE-OS-2 362 123 werden für Hexoprenalin Arzneiformen mit retardierter Wirkstofffreisetzung vorgeschlagen, bei denen der Wirkstoff in einem unverdaulichen bzw. unlöslichen Gerüst (Matrix), wie beispielsweise Natriumcarboxymethylcellulose oder Polymethacrylsäure, gleichmässig verteilt wird. Nach der Verabreichung solcher Gerüstarzneiformen wird dann der Wirkstoff während der Magen-Darm-Passage aus der Matrix durch Lösungs- und Diffusionsvorgänge wieder eluiert. Man erhält auf diese Weise zwar Präparate mit einer guten Verzögerungswirkung, aber eine genau definierte und in Abhängigkeit von der Ionenstärke im Gastrointestinaltrakt gleichmässig verzögerte Wirkstofffreigabe lässt sich mit Gerüstarzneiformen nicht erzielen.

Anderseits ist es seit langem üblich, saure oder basische Wirkstoffe zur Verlängerung der Wirkungsdauer an Kationen- oder Anionenaustauscherharze chemisch zu binden. Aus diesen Ionenaustauscherdepotformen oder Resinaten wird der Wirkstoff in Abhängigkeit von der Ionenstärke im Gastrointestinaltrakt verzögert freigesetzt. Die GB-PS-857 193 erteilt eine allgemeine Lehre zur Herstellung solcher Resinate, wobei über den Vernetzungsgrad des Ionenaustauscherharzes aber nur globale Angaben gemacht werden, die sich auf chemisch unterschiedliche Harzgruppen beziehen. Angaben über Ionenaustauscherharz-Wirkstoffkomplexe, in denen das Ionenaustauscherharz einen Vernetzungsgrad von 2 bis 5% aufweist, finden sich in dieser Druckschrift nicht.

Auch Phenylalkanolamine, wie Noradrenalin, Adrenalin und das Broncholytikum Isoprenalin wurden durch Umsetzung mit Kationen-Austauscherharzen in sogenannten «Retardformen» übergeführt, wobei als Ionentauscherharze auch Polystyrol diente, das mit Divinylbenzol vernetzt ist und Sulfonsäuregruppen enthält (CH-PS 379 057). Besonders bevorzugt wurden hierbei im Hinblick auf Beladungsfähigkeit und Wirkstoffabgabe solche Harze, die zu 7 bis 9% vernetzt sind. Harze mit niedrigem Vernetzungsgrad sind hiezu kaum geeignet, da der Wirkstoff zu schnell, z.B. innerhalb der ersten Stunde, fast vollständig freigegeben wird und somit keine ausreichende Retardierung vorliegt. Auch flüssige Arzneimittelzubereitungen, die ein Resinat des Wirkstoffes suspendiert enthalten, sind bereits vorgeschlagen worden, siehe die GB-PS 869 149. Auch hier wird für Phenylalkanolamine als Harzkomponente ein mit Divinylbenzol vernetztes Polystyrolharz, nämlich Zeocarb 225, vorgeschlagen, das einen Vernetzungsgrad von 8% besitzt.

Bei Anwendung dieser Methode auf Hexoprenalin zeigte sich aber, dass mit jenen Harzen, die z.B. bei Isoprenalin gute Eigenschaften ergaben, keine brauchbare Wirkstoffkombination erhalten werden konnte. Nicht nur, dass die Beladungskapazität der Harze mit 7 bis 9% Vernetzung unbefriedigend war, sie betrug für ein zu 8% vernetztes Harz z.B. nur 60 bis 65% derjenigen von Na, zeigten die Addukte bei längerem Stehen auch eine Braunfärbung, so dass sie für pharmazeutische Zwecke nicht geeignet waren.

Überraschenderweise konnte nun gefunden werden, dass auf Basis Hexoprenalin und Polystyrolen der oben angegebenen Art dann Zusammensetzungen mit guter Retardwirkung sowie auch guter Stabilität erhalten werden können, wenn für die Adduktbildung Harze mit nur 2 bis 5%, vorzugsweise rund 4% Vernetzung eingesetzt werden.

Gegenstand der vorliegenden Erfindung ist demnach eine Zusammensetzung mit verlängerter broncholytischer und tokolytischer Wirksamkeit, die dadurch gekennzeichnet ist, dass sie eine Verbindung von N,N'-Bis-[2-(3',4'-dihydroxyphenyl)-2-hydroxyäthyl]-hexamethylendiamin mit einem mit Divinylbenzol vernetzten Polystyrol mit Sulfonsäureresten als Ankergruppen mit einem Vernetzungsgrad von 2 bis 5% ist.

Bevorzugt werden Polystyrolharze mit einem Vernetzungsgrad von 4% eingesetzt, auch Harze mit einem Vernetzungsgrad von 4,5% sind sehr gut geeignet.

Die Beladungskapazität der erfindungsgemäss eingesetzten Polystyrolharze ist für Hexoprenalin über 90% der Kapazität von Natrium. Sie kann bei feinkrönigen, zu 4% vernetzten Harzen sogar 98% betragen. Grobkörnige Harze, z.B. solche einer Siebfraktion von 50 bis 100 mesh (US-Standard), zeigen eine etwas niedrigere Beladungskapazität als z.B. jene der gleichen Type einer Siebfraktion von 200 bis 400 mesh (50 bis 100 mesh 91,14% der Na-Kapazität, 200 bis 400 mesh 98,40% der Na-Kapazität), wobei der Effekt der Korngrösse nur bei dem Harz mit 4%iger Vernetzung so ausgeprägt ist, bei dem gleichen Harz aber mit nur 2%iger Vernetzung hingegen viel geringer ist (50 bis 100 mesh 91,46% der Na-Kapazität, 200 bis 400 mesh 93,96% der Na-Kapazität).

Die Freisetzungsrate der erfindungsgemässen Zusammensetzung ist sehr günstig. Bei in vitro durchgeführten Tests zeigte sich, dass bei Verwendung des erfindungsgemäss gekennzeichneten Polystyrols mit 4%iger Vernetzung nach 8 Stunden etwa 80% des ursprünglich vorhandenen Wirkstoffes Hexoprenalin freigesetzt werden. Verwendet man nur zu 2% vernetztes Harz, so ist meist schon nach etwa 3 Stunden eine Freisetzung von etwa 80% erreicht. Dass bei einem so wenig vernetzten Harz noch ein Rückhaltevermögen in diesem Ausmass zu sehen ist, ist als besonders überraschend zu werten. Die hohe Stabilität der erfindungsgemässen Zusammensetzung gegen Oxydation lässt auf eine gleichmässige und vollständige Bindung des Hexoprenalins am Harz schliessen.

Die Herstellung der erfindungsgemässen Zusammensetzung gelingt auf einfache Weise. Nachdem durch eine Säurevorbehandlung gewährleistet ist, dass das Harz mit Protonen beladen ist, wird der Wirkstoff durch Behandeln des Harzes mit einer wässrigen Lösung eines zweifachen Salzes von Hexoprenalin aufgebracht. Besonders geeignet ist hierzu eine wässrige Lösung des Dihydrochlorids von Hexoprenalin, aber auch andere Salze, wie z.B. das Dihydrobromid, das Disulfaminat und das Diacetat, sind hierzu geeignet.

Prinzipiell ist es möglich, die Behandlung des Harzes mit der wässrigen Lösung auf verschiedene Weise durchzuführen. So kann z.B. das Harz einfach mehrere Stunden lang mit einer wässrigen Lösung eines Salzes von Hexoprenalin geschüttelt werden, wobei durch $N_2$-Atmosphäre für Sauerstoffausschluss gesorgt wird und der Ansatz auch vor Licht zu schützen ist. Nach Abschluss der Beladung und Waschen mit Wasser kann das beladene Harz dann getrocknet und eingesetzt werden. Es ist aber auch nach der sog. Perkolationsmethode möglich, das Harz in einer Säule zu beladen, die mit der Salzlösung perkoliert wird. Auch hier ist für Sauerstoffausschluss und Lichtschutz während der Beladung zu sorgen.

Nach erfolgter Beladung sind die erfindungsgemässen Zusammensetzungen stabil. Sogar bei Lagerung in Sauerstoff-Atmosphäre bei einer Temperatur von 60 °C zeigen die Zusammensetzungen mit Harzen mit 4% Vernetzung auch nach 12 Wochen noch keine signifikante Veränderung.

Die erfindungsgemässen Zusammensetzungen werden in erster Linie zu Arzneimitteln für die perorale Verabreichung verarbeitet, wobei sie sowohl in fester Form, z.B. als Kapseln, Dragees oder Tabletten als auch in Suspensionen, z.B. als Sirup formuliert, vorliegen können. Eine Einzeldosis enthält vorzugsweise 2,4 mg der Zusammensetzung, das entspricht einer Einzeldosis von 1,5 mg Hexoprenalin.

Beispiel 1

Ein handelsübliches Polystyrolsulfonsäureharz mit 4%iger Vernetzung mit Divinylbenzol und einer Korngrösse von 200 bis 400 mesh wird zur Entfernung niedermolekularer Anteile hintereinander mit 2n Ammoniaklösung und 2n Salzsäure erhitzt, dekantiert und ausgewaschen. Es wird anschliessend zur Beladung mit Protonen mit 1n Salzsäure behandelt.

1 g des so vorbehandelten Harzes wird 10 Stunden lang mit einer Lösung von 1,5 g des Dihydrochlorides von N,N'-Bis-[2-(3',4'-dihydroxyphenyl)-2-hydroxyäthyl]-hexamethylendiamin in 100 ml bidestilliertem Wasser unter $N_2$-Atmosphäre und Lichtschutz geschüttelt. Nach Abnutschen des beladenen Harzes und Nachspülen mit bidestilliertem Wasser wird das Harz im Vakuumtrockenschrank getrocknet.

Die an das Harz gebundene, genaue Hexoprenalinmenge wird aus der Differenz zwischen der Ausgangskonzentration und der Restkonzentration in der Lösung nach Beladung ermittelt. Sie betrug 1250 mg entsprechend 5,07 mval, das sind 98,40% der Na-Kapazität des Harzes.

Die Freisetzungsrate des so hergestellten Resinates wurde nach der sogenannten Replacementclosed tube-methode bestimmt, das heisst, das Resinat wurde stündlich mit frischem Elutionsmittel versetzt und geschüttelt.

Als Elutionsflüssigkeit wurden in der ersten und zweiten Stunde künstlicher Magensaft (pH 1,3) und ab der dritten Stunde künstlicher Darmsaft (pH = 6,5) bei einer Temperatur von 37 °C verwendet.

Magensaft: 0,199 m; 2 g NaCl und 80 ml 1n HCl werden mit bidestilliertem Wasser zu 1000 ml aufgefüllt.

Darmsaft: 0,07 m; 10,09 g $Na_2HPO_4 \cdot H_2O$ und 1,76 g $NaH_2PO_4 \cdot 12H_2O$ werden mit bidestilliertem Wasser auf 1000 ml aufgefüllt.

Die dabei ermittelten Freisetzungsraten sind in folgender Tabelle angegeben:

| Stunden | Hexoprenalin % der Beladung |
|---|---|
| 1 | 36% |
| 2 | 55% |
| 3 | 66% |
| 4 | 70% |
| 5 | 75% |
| 6 | 78% |
| 7 | 80% |
| 8 | 81% |

Verwendet man anstelle des Harzes mit Korngrösse von 200 bis 400 mesh ein solches mit 50 bis 100 mesh, so beträgt die Beladung 4,24 mval/g bzw. 1048 mg/g, das sind 91,14% der Na-Kapazität.

Wird anstelle des Dihydrochlorids von N,N'-Bis-[2-(3',4'-dihydroxyphenyl)-2-hydroxyäthyl]-hexamethylendiamin das Disulfaminat, das Diacetat oder das Dihydrobromid eingesetzt und sonst wie oben angegeben verfahren, beträgt bei Verwendung des Harzes der Korngrösse 200 bis 400 mesh die Beladung bei Einsatz des Disulfaminats 4,98 mval/g, das sind 96,7%, bei Einsatz des Diacetats 4,92 mval/g, das sind 95,5% und bei Einsatz des Dihydrobromids 4,95 mval/g, das sind 96,1% der Na-Kapazität.

**Beispiel 2**

1 g eines nach der in Beispiel 1 gegebenen Vorschrift vorbehandelten Polystyrolsulfonsäureharzes mit 2%iger Vernetzung mit Divinylbenzol einer Korngrösse von 200 bis 400 mesh wird in 200 ml destilliertem Wasser aufgeschlämmt, in eine Säule eingetragen, dann werden mit der Säule 100 ml einer wässrigen Lösung von Hexoprenalin.2 HCl mit einer Hexoprenalinkonzentration von 1,5 Gew.-% unter N$_2$-Atmosphäre und Lichtschutz aufgegeben, wobei eine Tropfgeschwindigkeit von 1 ml/Minute angewendet wird. Anschliessend wird das bidestilliertem Wasser nachgespült, das Harz aus der Säule genommen und wie in Beispiel 1 getrocknet.

Die Beladung betrug 4,98 mval/g bzw. 1229,6 mg/g, das sind 93,96% der Na-Kapazität.

Die nach Beispiel 1 bestimmte Freisetzungsrate betrug:

| Stunden | Hexoprenalin % der Beladung |
|---------|------------------------------|
| 1 | 55% |
| 2 | 72% |
| 3 | 80% |
| 4 | 82% |
| 5 | 85% |
| 6 | 86% |
| 7 | 87% |
| 8 | 88% |

**Beispiel 3**

Aus einem nach Beispiel 1 hergestellten Hexoprenalin-Resinat wurde aus folgenden Bestandteilen eine Tablette hergestellt:

| | |
|---|---|
| Hexoprenalin-Resinat | 2,4 mg |
| Milchzucker | 95,6 mg |
| Maisstärke | 23,0 mg |
| Kollidon | 7,0 mg |
| Glycerinpalmitostearat | 2,0 mg |
| | 130,0 mg |

Die Tablette hat einen Wirkstoffgehalt von 1,5 mg Hexoprenalin.

Die so hergestellte Tablette kann auch mit Lack überzogen werden.

**Beispiel 4**

Aus den in Beispiel 3 genannten Bestandteilen wird ein Granulat hergestellt, das in eine Kapsel der Grösse 4 abgefüllt wird.

**Beispiel 5**

50,0 g Rohrzucker werden mit 48,54 g Aqua ad iniectabilia zu einem Sirup verkocht und am Ende des Kochprozesses mit 0,06 g p-Hydroxybenzoesäuremethylester und 0,03 g p-Hydroxybenzoesäurepropylester, beide gelöst in 1 ml Äthanol, versetzt. Nach Erkalten werden 1,2 g Methylcellulose und 0,15 g Cellulose mikrokristallin eingemengt und unter Einwirkung von Scherkräften eingearbeitet. Anschliessend werden 0,012 g des nach Beispiel 1 hergestellten Hexoprenalin-Resinats zugemischt. Man erhält 100 g Sirup, der für die perorale Verabreichung geeignet ist.

**Patentansprüche**

1. Zusammensetzung mit verlängerter broncholytischer und tokolytischer Wirksamkeit, die dadurch gekennzeichnet ist, dass sie eine Verbindung von N,N'-Bis-[2-(3',4'-dihydroxyphenyl)-2-hydroxyäthyl]-hexamethylendiamin mit einem mit Divinylbenzol vernetzten Polystyrol mit Sulfonsäureresten als Ankergruppen mit einem Vernetzungsgrad von 2 bis 5% ist.

2. Zusammensetzung nach Anspruch 1, dadurch gekennzeichnet, dass das Polystyrol einen Vernetzungsgrad von rund 4% besitzt.

3. Verfahren zur Herstellung der Zusammensetzung nach den Ansprüchen 1 und 2, dadurch gekennzeichnet, dass ein mit Protonen beladenes, mit Divinylbenzol vernetztes Polystyrol mit Sulfonsäureresten als Ankergruppen mit einem Vernetzungsgrad von 2 bis 5% in wässrigem Medium unter Lichtschutz und in Stickstoff-Atmosphäre mit einer wässrigen Lösung eines Bis-Salzes des Bis-[2-(3',4'-dihydroxyphenyl)-2-hydroxyäthyl]-hexamethylendiamins behandelt wird.

4. Broncholytisch und tokolytisch wirkendes Mittel mit verlängerter, gleichmässiger Wirkstoffabgabe, dadurch gekennzeichnet, dass es als Wirkstoff die Zusammensetzung gemäss den Patentansprüchen 1 und 2 enthält.

**Claims**

1. Composition which has a prolonged broncholytic and tocolytic activity, characterised in that it is a compound of N,N'-bis-[2-(3',4'-dihydroxyphenyl)-2-hydroxy-ethyl]-hexamethylenediamine with a polystyrene which is crosslinked with divinylbenzene, contains sulphonic acid radicals as anchor groups and has a degree of crosslinking of 2 to 5%.

2. Composition according to Claim 1, characterised in that the polystyrene has a degree of crosslinking of 4%.

3. Process for the preparation of the composition according to Claims 1 and 2, characterised in that a polystyrene which is crosslinked with divinylbenzene, contains sulphonic acid radicals as anchor groups and has a degree of crosslinking of 2 to 5% and has been charged with protons, is treated in an aqueous medium, with protection against light and in a nitrogen atmosphere, with an aqueous solution of a bis-salt of bis-[2-(3',4'-dihydroxyphenyl)-2-hydroxyethyl]-hexamethylene-diamine.

4. An agent which has a broncholytic and tocolytic action and releases the active ingredient over a prolonged period and at a uniform rate, characterised in that it contains the composition according to Claims 1 and 2 as the active ingredient.

**Revendications**

1. Composition ayant une activité broncholytique et tocolytique prolongée, caractérisée en ce qu'elle est constituée par une combinaison de N,N'-bis-[2-(3',4'-dihydroxyphényl)-2-hydroxy- éthyl]-hexaméthylènediamine avec un polystyrène à groupes acide sulfonique servant de groupes de fixation réticulé par du divinylbenzène, ayant un degré de réticulation de 2 à 5%.

2. Composition suivant la revendication 1, caractérisée en ce que le polystyrène présente un degré de réticulation de 4% environ.

3. Procédé pour la préparation de la composition suivant les revendications 1 et 2, caractérisé en ce qu'on traite un polystyrène à groupes acide sulfonique servant de groupes de fixation, réticulé par du divinylbenzène et chargé de protons, ayant un degré de réticulation de 2 à 5%, dans un milieu aqueux à l'abri de la lumière et dans une atmosphère d'azote, avec une solution aqueuse d'un disel de bis-[2-(3',4'-dihydroxyphényl)-2-hydroxyéthyl]-hexaméthylènediamine.

4. Médicament à effet broncholytique et tocolytique assurant une libération régulière et prolongée de substance active, caractérisé en ce qu'il renferme, à titre de substance active, la composition suivant les revendications 1 et 2.